# EUROPÄISCHE PATENTSCHRIFT

(11) **EP 1 669 374 B1**
(45) Veröffentlichungstag und Bekanntmachung des Hinweises auf die Patenterteilung: **21.11.2012**
(21) Anmeldenummer: 05026644.4
(22) Anmeldetag: 06.12.2005
(51) Int. Cl.: C08F 2/16, C08F 2/06, C08F 2/00, A61K 8/00, A61K 9/00, A61K 8/04, A61K 8/81, A61K 47/32, A61Q 19/00, C08F 226/10

(54) **Verfahren zur Herstellung wässriger Sekundärdispersionen von in Wasser nicht löslichen Polymeren**
Method for forming aqueous secondary dispersions of water insoluble polymers
Procédé pour préparation de dispersions aqueuses secondaires de polymères insolubles dans l'eau

(30) Priorität: 07.12.2004 DE 102004058952
(43) Veröffentlichungstag der Anmeldung: 14.06.2006
(73) Patentinhaber: BASF SE, 67056 Ludwigshafen (DE)
(72) Erfinder: Bouillo, Nathalie, 76532 Baden-Baden (DE); Rössler, Gerhard, 67141 Neuhofen (DE)

(56) Entgegenhaltungen:
- DE-A1- 4 139 963
- US-A- 5 395 904

## Beschreibung

Die vorliegende Erfindung betrifft ein Verfahren zur Herstellung wässriger Sekundärdispersionen von wasserunlöslichen Copolymeren durch radikalische Lösungspolymerisation ethylenisch ungesättigter Monomere M, umfassend:
i) 10 bis 90 Gew-% bezogen auf die Gesamtmonomermenge M wenigstens eines monoethylenisch ungesättigten Monomers M1 mit einer Wasserlöslichkeit von nicht mehr als 30 g/l bei 25 °C und 1 bar und
ii) wenigstens ein hydrophiles, monoethylenisch ungesättigtes, nichtionisches Comonomere M2 mit einer Wasserlöslichkeit von wenigstens 80 g/l bei 25 °C und 1 bar,
in einem organischen, mit Wasser mischbaren Lösungsmittel oder einer Mischung von Wasser mit einem organischen, mit Wasser mischbaren Lösungsmittel als Polymerisationsmedium und anschließendes Ersetzen des organischen Lösungsmittels durch Wasser. Die vorliegende Erfindung betrifft auch die durch das erfindungsgemäße Verfahren erhältlichen wässrigen Polymerdispersionen und deren Verwendung insbesondere in der Pharmazie und der Kosmetik.

Wässrige Dispersionen von in Wasser unlöslichen Polymeren werden vielfach durch radikalische wässrige Emulsionspolymerisation ethylenisch ungesättigter Monomere in Gegenwart oberflächenaktiver Substanzen wie Schutzkolloide und Emulgatoren hergestellt. Die radikalische wässrige Emulsionspolymerisation findet ihre Grenzen, wenn die zu polymerisierenden Monomere neben den üblicherweise verwendeten Monomeren mit begrenzter Wasserlöslichkeit größere Mengen an hydrophilen Monomeren mit einer guten Wasserlöslichkeit, z. B. wenigstens 80 g/l bei 25 °C und 1 bar, umfassen. Hier tritt als Konkurrenzreaktion die Homopolymerisation der Monomere mit hoher Wasserlöslichkeit auf.

Als Alternative bietet sich die Polymerisation derartiger Monomere oder Monomermischungen in einem organischen Lösungsmittel, welches die zu polymerisierenden Monomeren vollständig zu lösen vermag, an. Nach Ersatz des organischen Lösungsmittels durch Wasser erhält man eine wässrige Dispersion dieser Polymerisate. Die so hergestellten wässrigen Dispersionen werden im Unterschied zu den durch Emulsions- oder Suspensionspolymerisation hergestellten Primärdispersionen auch als Sekundärdispersionen bezeichnet. Zur Stabilisierung wässriger Sekundärdispersionen, d.h. zur Stabilisierung der Polymerteilchen in der Sekundärdispersion, ist es allerdings erforderlich, vor oder während des Ersatzes des organischen Lösungsmittels durch Wasser oberflächenaktive Substanzen, insbesondere Emulgatoren, zuzugeben. Der Einsatz derartiger oberflächenaktiver Substanzen ist jedoch in vielen Anwendungsbereichen, beispielsweise in der Kosmetik und in der Pharmazie, nicht erwünscht.

Verschiedentlich wurde Polyvinylpyrrolidon zur Stabilisierung der Polymerteilchen bei einer Emulsions- oder Suspensionspolymerisation hydrophober Monomere in einem polaren Medium, beispielsweise in Wasser oder Wasser/C₁-C₄-Alkanol-Mischungen, vorgeschlagen (siehe z. B. Korea Polymer Journal 6 (1998), S. 405 - 413, J. Polymer Sci., Part A: Polymer Chemistry 34 (1996), S. 1857 - 1871 und J. Polymer Sci., Part A: Polymer Chemistry 32 (1994), S. 1087 -1100).

Die US 5,395,904 A beschreibt einen Verfahren zur Herstellung homogener Copolymere von Vinylpyrrolidon und Vinylacetat. Diese bilden klare wässrige Lösungen und haben einen hohen Trübungspunkt.

Die DE 4139963 A1 beschreibt ein Verfahren zur Herstellung eines redispergierbaren, rieselfähigen Dispersionspulvers aus einem 15 bis 20 Gew.-% Vinylpyrrolidon einpolymerisiert enthaltenden Vinylpyrrolidon-Vinylacetat-Copolymer. Die Mischung der Monomere wird in organischer Lösung copolymerisiert. Die so entstandene Lösung wird nach Zusatz eines Tensides einem Lösungsmittelaustausch gegen Wasser unterworfen und die so erhaltene Dispersion wird sprüh- oder gefriergetrocknet.

Eigene Untersuchungen der Erfinder haben allerdings gezeigt, dass sich durch Zusatz von Polyvinylpyrrolidon während oder im Anschluss an die Polymerisation hydrophober Monomere keine stabilen wässrigen Sekundärdispersionen erhalten lassen.

Der vorliegenden Erfindung liegt daher die Aufgabe zu Grunde, ein Verfahren zur Herstellung wässriger Sekundärdispersionen von wasserunlöslichen Polymeren, die durch radikalische Polymerisation ethylenisch ungesättigter Monomere M gemäß dem eingangs definierten Verfahren hergestellt wurden, bereitzustellen.

Es wurde überraschenderweise gefunden, dass diese Aufgabe dadurch gelöst werden kann, dass man gegen Ende der Polymerisation einen Teil der Monomere M in Form einer Monomerzusammensetzung M' zugibt, die als Monomere im wesentlichen nur Monomere M2 enthält.

Gegenstand der vorliegenden Erfindung ist daher ein Verfahren zur Herstellung wässriger Sekundärdispersionen von wasserunlöslichen Copolymeren, umfassend
a) radikalische Lösungspolymerisation ethylenisch ungesättigter Monomere M, die:
   i. 10 bis 90 Gew-%, bezogen auf die Gesamtmonomermenge M, wenigstens eines monoethylenisch ungesättigten Monomers M1 mit einer Wasserlöslichkeit von nicht mehr als 30 g/l bei 25 °C und 1 bar und
   ii. 10 bis 90 Gew-%, bezogen auf die Gesamtmonomermenge M, wenigstens eines hydrophilen, monoethylenisch ungesättigten, nichtionischen Comonomeres M2 mit einer Wasserlöslichkeit von wenigstens 80 g/l bei 25 °C und 1 bar umfassen,
   in einem organischen, mit Wasser mischbaren Lösungsmittel, das bei 25 °C und 1 bar mit 50 Gewichteilen Wasser, bezogen auf 100 Gewichtsteile organisches Lösungsmittel, eine homogene Phase bildet, oder in einer Mischung von Wasser mit dem organischen, mit Wasser mischbaren Lösungsmittel als Polymerisationsmedium und
b) Ersetzen des organischen Lösungsmittels durch Wasser,
dadurch gekennzeichnet, dass man 0,5 bis 20 Gew.-% der Monomere M bezogen auf die Gesamtmenge der zu polymerisierenden Monomere M in Form einer Monomerzusammensetzung M', die als Monomere zu mehr als 90 Gew.-% Monomere M2 enthält, zu einem Zeitpunkt zugibt, wenn wenigstens 80% desjenigen Anteils der Monomere M, der von M' verschieden ist, sich bereits im Polymerisationsgefäß unter Polymerisationsbedingungen befindet.

Die Erfindung ist mit einer Reihe von Vorteilen verbunden. Zum einen sind die erfindungsgemäß erhältlichen wässrigen Sekundärdispersionen auch bei längerer Lagerung stabil, d. h. sie zeigen keine oder nur sehr geringe Anzeichen an Koagulation und/oder Sedimentation. Außerdem ist der Einsatz oberflächenaktiver Substanzen, insbesondere Emulgatoren, zur Herstellung der erfindungsgemäßen wässrigen Sekundärdispersionen nicht erforderlich. Die erfindungsgemäß erhältlichen wässrigen Polymerdispersionen sind daher in besonderem Maße für solche Anwendungen geeignet, bei denen die in einer normalen wässrigen Emulsionspolymerisation üblicherweise verwendeten grenzflächenaktiven Substanzen stören oder aus sonstigen Gründen nicht erwünscht sind, wie beispielsweise in kosmetischen oder pharmazeutischen Zubereitungen.

Daher betrifft ein weiterer Gegenstand der vorliegenden Erfindung die nach dem erfindungsgemäßen Verfahren erhältlichen wässrigen Sekundärdispersionen und die daraus durch Trocknung gewonnenen Polymere. Die Erfindung betrifft außerdem die Verwendung der so erhältlichen Sekundärdispersionen und Polymere zur Herstellung kosmetischer oder pharmazeutischer Zubereitungen. Die Erfindung betrifft weiterhin die Verwendung der aus den Sekundärdispersionen erhältlichen Pulver zur Herstellung kosmetischer oder pharmazeutischer Zubereitungen.

Erfindungsgemäß umfassen die zu polymerisierenden Monomere M wenigstens ein monoethylenisch ungesättigtes, hydrophobes Monomer M1. Hierunter versteht man Monomere mit einer Wasserlöslichkeit von nicht mehr als 30 g/l, insbesondere nicht mehr als 10 g/l und speziell nicht mehr als 1 g/l bei 25 °C und 1 bar.

Die Monomere M1 machen wenigstens 10 Gew.-%, häufig wenigstens 15 Gew.-% und insbesondere wenigstens 20 Gew.-% der Monomere M aus, um eine hinreichende Wasserunlöslichkeit des erhaltenen Polymerisats zu gewährleisten. Ihr Anteil an der Gesamtmonomermenge M wird 90 Gew.-%, häufig 80 Gew.-% und insbesondere 70 Gew.-% nicht überschreiten. Dementsprechend beträgt der Anteil der Monomere M2 an der Gesamtmenge der Monomere M wenigstens 10 Gew.-%, häufig wenigstens 20 Gew.-% und insbesondere wenigstens 30 Gew.-% und wird 90 Gew.-%, häufig 85 Gew.-% und speziell 80 Gew.-% nicht überschreiten. In einer bevorzugten Ausführungsform der Erfindung beträgt der Anteil der Monomere M1 10 bis 60 Gew.-%, insbesondere 15 bis 50 Gew.-% und speziell 20 bis 40 Gew.-%, bezogen auf die Gesamtmenge der Monomere M. Dementsprechend beträgt in dieser Ausführungsform der Anteil der Monomere M 2 an der Gesamtmenge der Monomere M 40 bis 90 Gew.-%, insbesondere 50 bis 85 Gew.-% und speziell 60 bis 80 Gew.-%.

Zu den Monomeren M1 zählen Ester monoethylenisch ungesättigter C₃-C₈₋Carbonsäuren deren Alkoholkomponente ein Kohlenwasserstoffrest mit z.B. 1 bis 30 Kohlenstoffatomen ist, ausgenommen Methylacrylat, weiterhin Vinylester und Allylester aliphatischer Carbonsäuren mit 2 bis 30 C-Atomen, Olefine mit 2 bis 20 C-Atomen, Vinylaromaten wie Styrol und alkylsubstituierte Styrole wie α-Methylstyrol und tert. Butylstyrol, weiterhin N-substituierte Amide und N,N-disubstituierte Amide monoethylenisch ungesättigter C₃-C₈-Carbonsäuren, deren Substituent(en) am Stickstoff 1 bis 30 C-Atome aufweist.

Insbesondere eignet sich das erfindungsgemäße Verfahren zur Polymerisation von Monomeren M, worin die Monomere M1 wenigstens ein Monomer M1a umfassen, das wenigstens einen Kohlenwasserstoffrest mit 6 bis 30 C-Atomen und insbesondere mit 8 bis 24 C-Atomen aufweist. In einer bevorzugten Ausführungsform machen die Monomere M1a wenigstens 50 Gew.-% der Monomere M1 und insbesondere wenigstens 80 Gew.-% der Monomere M1 aus. Insbesondere sind die Monomere M1a alleiniges Monomer M1.

Zu Kohlenwasserstoffresten mit 6 bis 30 und insbesondere 8 bis 24 C-Atomen zählen lineares oder verzweigtes Alkyl, das eine oder zwei Doppelbindungen aufweisen kann, im Folgenden auch C₆-C₃₀-Alkyl, C₆-C₃₀-Alkenyl, C₆-C₃₀-Alkadienyl, bzw. C₈-C₂₄-Alkyl, C₈-C₂₄-Alkenyl oder C₈-C₂₄-Alkadienyl, z. B. n-Hexyl, n-Octyl, 2-Ethylhexyl, n-Decyl, 2-Propylheptyl, Lauryl, Myristyl, Cetyl, Stearyl, Oleyl und Behenyl, weiterhin cycloaliphatische Reste, die gegebenenfalls 1, 2, 3 oder 4 C₁-C₆-Alkylgruppen aufweisen und die insgesamt 6 bis 30 und insbesondere 8 bis 24 C-Atome aufweisen (im Folgenden C₆₋C₃₀-Cycloalkyl) wie Cyclohexyl, Cycloheptyl, 4-tert.-Butyl, Norbornyl, Adamantyl, Tricyclo[3.2.1.0]decyl und 3,3,5,5-Tetramethylcyclohexyl sowie weiterhin lineares oder verzweigtes Alkyl, das ein oder zwei Cycloalkylreste trägt und insgesamt 6 bis 30 und insbesondere 8 bis 24 C-Atome aufweist, beispielsweise Cyclohexylmethyl, Cycloheptylmethyl, 2-Cyclohexylethyl, Bis(cyclohexyl)methyl, 2,2-Bis(cyclohexyl)ethyl und 3,3-Bis(cyclohexyl)butyl.

Zu den Monomeren M1a zählen Ester monoethylenisch ungesättigter Carbonsäuren, insbesondere solcher mit 3 bis 8 C-Atomen und die Amide derartiger monoethylenisch ungesättigter Carbonsäuren, wie Acrylsäure oder Methacrylsäure, die wenigstens einen der vorgenannten Kohlenwasserstoffreste mit 6 bis 30 und insbesondere 8 bis 24 C-Atome aufweisen.

Zu den Monomeren M1a zählen auch Vinylester und Allylester von aliphatischen Carbonsäuren mit 6 bis 31 und insbesondere mit 9 bis 25 C-Atomen, beispielsweise Vinylhexanoat, Vinyloctanoat, Vinyllaurat, Vinylmyristat, Vinylstearat, Vinyloleat und Vinylbehenat.

Zu den Monomeren M1a zählen außerdem Olefine mit wenigstens 6 C-Atomen und alkylsubstituiertes Styrol, z.B. tert.-Butylstyrol.

Unter den Monomeren M1a sind Ester monoethylenisch ungesättigter Carbonsäuren, insbesondere von Monocarbonsäuren mit 3 bis 8 C-Atomen und die Amide derartiger monoethylenisch ungesättigter Carbonsäuren, insbesondere von Monocarbonsäuren mit 3 bis 8 C-Atomen, die wenigstens einen der vorgenannten Kohlenwasserstoffreste mit 6 bis 30 und insbesondere mit 8 bis 24 C-Atome aufweisen, bevorzugt. Insbesondere bevorzugt sind die Ester der Acrylsäure mit C₈-C₂₄-Alkanolen oder C₈-C₂₄₋Cycloalkanolen, Ester der Methacrylsäure mit C₈-C₂₄-Alkanolen oder C₈-C₂₄₋Cycloalkanolen, N-C₈-C₂₄-Alkylamide und die N-C₈-C₂₄-Cycloalkamide der Acrylsäure und der Methacrylsäure sowie die N,N-Bis-(C₈-C₂₄-Alkyl)amide der Acrylsäure und der Methacrylsäure. Besonders bevorzugt sind die vorgenannten Ester der Acrylsäure und der Methacrylsäure mit C₈-C₂₄-Alkanolen wie n-Octylacrylat, 2-Ethylhexylacrylat, n-Decylacrylat, 2-Propylheptylacrylat, Laurylacrylat, Myristylacrylat, Cetylacrylat, Stearylacrylat, Oleylacrylat und Behenylacrylat, n-Hexylmethacrylat, n-Octylmethacrylat, 2-Ethylhexylmethacrylat, n-Decylmethacrylat, 2-Propylheptylmethacrylat, Laurylmethacrylat, Myristylmethacrylat, Cetylmethacrylat, Stearylmethacrylat, Oleylmethacrylat und Behenylmethacrylat. Bevorzugt sind auch die N-C₈-C₂₄-Alkylamide der Acrylsäure und der Methacrylsäure wie n-Octylacrylamid, 2-Ethylhexylacrylamid, n-Decylacrylamid, 2-Propylheptylacrylamid, Laurylacrylamid, Myristylacrylamid, Cetylacrylamid, Stearylacrylamid, Oleylacrylamid und Behenylacrylamid, n-Hexylmethacrylamid, n-Octylmethacrylamid, 2-Ethylhexylmethacrylamid, n-Decylmethacrylamid, 2-Propylheptylmethacrylamid, Laurylmethacrylamid, Myristylmethacrylamid, Cetylmethacrylamid, Stearylmethacrylamid, Oleylmethacrylamid und Behenylmethacrylamid.

Zu den Monomeren M1 zählen weiterhin monoethylenisch ungesättigte Monomere M1b mit einer begrenzten Wasserlöslichkeit im Bereich von > 1 g/l bis 30 g/l. Hierzu zählen beispielsweise die C₁-C₄-Alkylester von monoethylenisch ungesättigten Carbonsäuren mit 3 bis 8 C-Atomen, insbesondere die Ester der Acrylsäure und der Methacrylsäure wie Ethylacrylat, n-Propylacrylat, Isopropylacrylat, n-Butylacrylat und tert.-Butylacrylat, weiterhin Methylmethacrylat, Ethylmethacrylat, n-Propylmethacrylat, lsopropylmethacrylat, n-Butylmethacrylat und tert.-Butylmethacrylat, aber auch Vinylester aliphatischer Carbonsäuren mit 2 bis 4 C-Atomen wie Vinylacetat, Vinylpropionat und Vinylbutyrat.

In einer besonders bevorzugten Ausführungsform der Erfindung umfassen die Monomere M 10 bis 60 Gew.-%, insbesondere 15 bis 50 Gew.-% und speziell 20 bis 40 Gew.-% Monomere M1a und 40 bis 90 Gew.-%, insbesondere 50 bis 85 Gew.-% und 60 bis 80 Gew.-% Monomere M2, jeweils bezogen auf das Gesamtgewicht der zu Polymerisierenden Monomere M. In einer anderen ebenfalls bevorzugten Ausführungsform umfassen die Monomere M 5 bis 50 Gew.-%, insbesondre 10 bis 40 Gew.-% Monomere M1a, 10 bis 60 Gew.-%, insbesondere 10 bis 50 Gew.-% Monomere M1b und 20 bis 85, insbesondere 45 bis 80 Gew.-% Monomere M2, jeweils bezogen auf das Gesamtgewicht der zu polymerisierenden Monomere M.

Zu den Monomeren M2 zählen grundsätzlich alle nichtionischen monoethylenisch ungesättigten Monomere mit einer Wasserlöslichkeit von wenigstens 80 g/l bei 25 °C und 1 bar. Hierzu zählen beispielsweise
- Hydroxy-C₂-C₄-alkylester monoethylenisch ungesättigter Mono- und Di-C₃-C₈₋carbonsäuren, insbesondere die Hydroxy-C₂-C₄-alkylester der Acrylsäure und der Methacrylsäure wie 2-Hydroxyethylacrylat, 3-Hydroxypropylacrylat, 2-Hydroxypropylacrylat, 4-Hydroxybutylacrylat, 2-Hydroxyethylmethacrylat, 3-Hydroxypropylmethacrylat, 2-Hydroxypropylmethacrylat und 4-Hydroxybutylmethacrylat;
- N-Vinyllactame, insbesondere solche mit 5 bis 8 Ringatomen wie N-Vinylpyrrolidon, N-Vinylpiperidon, N-Vinylmorpholinon und N-Vinylcaprolactam;
- N-Vinylamide aliphatischer Carbonsäuren mit 1 bis 6 und insbesondere 1 bis 4 C-Atomen wie N-Vinylformamid, N-Vinylacetamid und N-Vinylpropionamid;
- Amide, Hydrox-C₁-C₄-alkylamide und C₁-C₄-Alkyloxy-C₁-C₄-alkylamide monoethylenisch ungesättigter C₃-C₈-Monocarbonsäuren wie Acrylamid, Methacrylamid, N-(Methoxymethyl)(meth)acrylamid, N-(Ethoxymethyl)(meth)acrylamid, N-(2-Methoxyethyl)(meth)acrylamid, N-(2-Ethoxyethyl)(meth)acrylamid und dergleichen;
- monoethylenisch ungesättigte Monomeren mit Polyethergruppen, insbesondere mit Poly-C₂-C₄-alkylenoxidgruppen und speziell mit Polyethylenoxid-Gruppen,
wobei die Polyethergruppen vorzugsweise ein Molekulargewicht (Zahlenmittel) im Bereich von 100 bis 5000 aufweisen. Hierzu zählen insbesondere die Vinyl- und Allylether von Poly-C₂-C₄-alkylenglykolen sowie die Monoester monoethylenisch ungesättigter C₃-C₈-Mono- und C₄-C₈-Dicarbonsäuren mit Poly-C₂-C₄₋alkylenglykolen, insbesondere die Acrylsäure- und die Methacrylsäuremonoester derartiger Poly-C₂-C₄-alkylenglykole
- vinyl-substituierte Stickstoffheteroaromaten wie 2-, 3- und 4-Vinylpyridin und N-Vinylimidazol;
- monoethylenisch ungesättigte, Harnstoffgruppen tragende Monomere wie N-(2-Acrylamidoethyl)-imidazolin-2-on und N-(2-Methacrylamidoethyl)-imidazolin-2-on;
- Aldehyd- oder Ketogruppen aufweisende Ester und Amide der vorgenannten monoethylenisch ungesättigten Carbonsäuren wie 3-(Acrylamido)-3-methylbutan-2-on (Diacetonacrylamid), 3-(Methacrylamido)-3-methylbutan-2-on, 2,4-Dioxapentylacrylat und 2,4-Dioxapentylmethacrylat; und
- monoethylenisch ungesättigte Monomere mit einer primären, sekundären oder tertiären Aminogruppe, insbesondere Monomere der allgemeinen Formel I 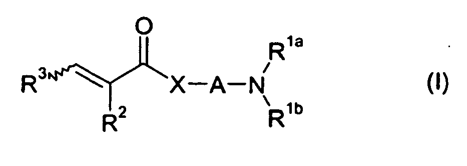
worin X für Sauerstoff oder eine Gruppe N-R⁴ steht;
- A: für C₂-C₈-Alkylen, z. B. 1,2-Ethandiyl, 1,2- oder 1,3-Propandiyl, 1,4-Butandiyl oder 2-Methyl-1,2-propandiyl, das gegebenenfalls durch 1, 2 oder 3 nicht benachbarte Sauerstoffatome unterbrochen ist, wie in 3-Oxapentan-1,5-diyl, steht
- R^{1a}, R^{1b}: unabhängig voneinander für Wasserstoff, C₁-C₁₀-Alkyl, C₅-C₁₀-Cycloalkyl, Phenyl oder Phenyl-C₁-C₄-alkyl stehen und insbesondere beide gemeinsam C₁-C₄-Alkyl bedeuten;
- R²: Wasserstoff oder C₁-C₄-Alkyl, insbesondere Wasserstoff oder Methyl, bedeutet;
- R³: Wasserstoff oder C₁-C₄-Alkyl bedeutet insbesondere Wasserstoff; und
- R⁴: Wasserstoff oder C₁-C₄-Alkyl und insbesondere Wasserstoff bedeutet.
Beispiele für Monomere der Formel I sind 2-(N,N-Dimethylamino)ethylacrylat, 2-(N,N-Dimethylamino)ethylmethacrylat, 2-(N,N-Dimethylamino)ethylacrylamid, 3-(N,N-Dimethylamino)propylacrylamid, 3-(N,N-Dimethylamino)propylmethacrylamid und 2-(N,N-Dimethylamino)ethylmethacrylamid.

In einer bevorzugten Ausführungsform sind die Monomere M2 unter den vorgenannten N-Vinyllactamen mit 5 bis 8 Ringatomen sowie den N-Vinylamiden aliphatischer Carbonsäuren mit 1 bis 6 und insbesondere 1 bis 4 C-Atomen ausgewählt. Besonders bevorzugte Monomere M2 sind die vorgenannten N-Vinyllactame und insbesondere N-Vinylpyrrolidon.

Gegebenenfalls können die zu polymerisierenden Monomere M auch bis zu 20 Gew.-% und insbesondere bis 10 Gew.-%, z. B. 0,05 bis 10 Gew.-%, Monomere M3 umfassen, die von den Monomeren M1 und M2 verschieden sind. Zu den Monomeren M3 zählen monoethylenisch ungesättigte Monomere M3.s, die wenigstens eine Säuregruppe oder wenigstens eine anionische Gruppen aufweisen, insbesondere Monomere, die eine Sulfonsäuregruppe, eine Phosphonsäuregruppe oder ein oder zwei Carbonsäuregruppen aufweisen, sowie die Salze derartiger Monomere, insbesondere die Alkalimetallsalze, z. B. die Natrium- oder Kaliumsalze sowie die Ammoniumsalze. Hierzu zählen ethylenisch ungesättigte Sulfonsäuren, insbesondere Vinylsulfonsäure, 2-Acrylamido-2-methylpropansulfonsäure, 2-Acryloxyethansulfonsäure und 2-Methacryloxyethansulfonsäure, 3-Acryloxy- und 3-Methacryloxypropansulfonsäure, Vinylbenzolsulfonsäure und deren Salze, ethylenisch ungesättigte Phosphonsäuren, wie Vinylphosphonsäure und Vinylphosphonsäuredimethylester und deren Salze und α,β-ethylenisch ungesättigte C₃-C₈-Mono- und C₄-C₈-Dicarbonsäuren, insbesondere Acrylsäure, Methacrylsäure, Crotonsäure, Maleinsäure, Fumarsäure und Itaconsäure. Der Anteil der Monomere M3.s wird häufig nicht mehr als 10 Gew.-%, z. B. 0,05 bis 10 Gew.-% und insbesondere 0,1 bis 5 Gew.-%, bezogen auf die Gesamtmenge der Monomere M, ausmachen. In einer bevorzugten Ausführungsform umfassen die Monomere M keine oder nicht mehr als 0,1 Gew.-% Monomere M3.s.

Zu den Monomeren M3 zählen weiterhin monoethylenisch ungesättigte Monomere M3.k, die wenigstens eine kationische Gruppe aufweisen. Zu den Monomeren M3.k zählen insbesondere solche, die eine quartäre Ammoniumgruppe oder eine quaternisierte Iminogruppe aufweisen. Beispiele für Monomere mit einer quaternisierten Iminogruppe sind N-Alkylvinylpyridiniumsalze und N-Alkyl-N'-vinylimidazoliniumsalze wie N-Methyl-N'-vinylimidazoliniumchlorid oder Metosulfat sowie weiterhin ω-(Tri-C₁-C₄₋alkylammonium)-C₂-C₄-alkylacrylate, ω-(Tri-C₁-C₄-alkylammonium)-C₂-C₄₋alkylmethacrylate, ω-(Tri-C₁-C₄-alkylammonium)-C₂-C₄-alkylacrylamide und ω-(Tri-C₁₋C₄-alkylammonium)-C₂-C₄-alkylmethacrylamide wie 2-(N,N,N-Trimethylammonium)-ethylacrylat-Chlorid, 2-(N,N,N-Trimethylammonium)ethylmethacrylat-Chlorid, 2-(N,N,N-Trimethylammonium)ethylmethacrylamid-Chlorid, 3-(N,N,N-Trimethylammonium)propylacrylamid-Chlorid, 3-(N,N,N-Trimethylammonium)propyl-methacrylamid-Chlorid, 2-(N,N,N-Trimethylammonium)ethylacrylamid-Chlorid, sowie die entsprechenden Metosulfate und Sulfate.

Der Anteil der Monomere M3.k an den Monomeren M beträgt vorteilhafterweise nicht mehr als 20 Gew.-%, z.B. 0,1 bis 20 Gew.-%, insbesondere 0,1 bis 15 Gew.-%, und speziell 1 bis 10 Gew.-%. In einer bevorzugten Ausführungsform umfassen die Monomere M keine oder nicht mehr als 0,1 Gew.-% Monomere M3.k.

Erfindungsgemäß sind die in der Monomerzusammensetzung M' enthaltenen Monomere M2 im wesentlichen alleiniger Monomerbestandteil von M', d.h. der Anteil der Monomere M2 beträgt mehr als 90 Gew.-% und insbesondere mehr als 99 Gew.-% aller in der Monomerzusammensetzung M' enthaltenen Monomere. Geringe Mengen anderer Monomere sind jedoch nicht von Nachteil. Die Monomerzusammenseztung M' kann den Monomerbestandteil in unverdünnter Form oder in gelöster oder verdünnter Form enthalten. Geeignete Lösungsmittel sind neben Wasser die für die Polymerisation eingesetzten organischen Lösungmittel und deren Mischungen mit Wasser. Vorzugsweise liegt die Monomerkonzentration in der Monomerzusammensetzung bei wenigstens 50 Gew.-%, insbesondere wenigstens 70 Gew.-%.

Erfindungsgemäß können die in dem verbleibenden Anteil der Monomere M enthaltenen Monomeren M2 von den in der Monomerzusammesetzung M' enthaltenen Monomere M2 verschieden oder identisch mit ihnen sein. Häufig wird es sich jedoch bei den Monomeren M2 in der Monomerzusammensetzung M' um die gleiche Art an Monomeren wie in der verbleibenden Menge der Monomere M handeln.

Für das erfindungsgemäße Verfahren ist es von Vorteil, wenn die in der Monomerzusammensetzung M' enthaltenen Monomere M2 unter den vorgenannten N-Vinyllactamen mit 5 bis 8 Ringatomen sowie den N-Vinylamiden aliphatischer Carbonsäuren mit 1 bis 6 und insbesondere 1 bis 4 C-Atomen ausgewählt sind. Besonders bevorzugte Monomere M2 sind die vorgenannten N-Vinyllactame und insbesondere N-Vinylpyrrolidon sowie Mischungen der vorgenannten Monomere M2 mit N-Vinyllactamen und speziell mit N-Vinylpyrrolidon, die, bezogen auf die Gesamtmenge der Monomere M2, bis zu 50 Gew.-%, z. B. 1 bis 50 Gew.-%, insbesondere jedoch nicht mehr als 30 Gew.-% und besonders bevorzugt nicht mehr als 10 Gew.-% von N-Vinylpyrrolidon verschiedene Monomere M2 enthalten. Insbesondere ist N-Vinylpyrrolidon alleiniges Monomer M'

Der über die Monomerzusammensetzung M' zugegebene Anteil der Monomere M2 beträgt in der Regel 1 bis 50 Gew.-%, insbesondere 2 bis 30 Gew.-% und speziell 5 bis 20 Gew.-% der Gesamtmenge der zu polymerisierenden Monomere M2.

Die über die Monomerzusammensetzung M' eingebrachte Monomeremenge (Monomere M') beträgt wenigstens 0,5 Gew.-% und wird 20 Gew.-%, bezogen auf die Gesamtmenge der zu polymerisierenden Monomere M, nicht überschreiten. Vorzugsweise liegt sie im Bereich von 1 bis 20 Gew.-%, insbesondere im Bereich von 3 bis 15 Gew.-% und besonders bevorzugt im Bereich von 5 bis 13 Gew.-%, bezogen auf die Gesamtmenge der zu polymerisierenden Monomere M.

Die Polymerisation der Monomere M erfolgt unter den für eine radikalische Polymerisation in einem organischen Lösungsmittel üblichen Bedingungen in Gegenwart von Radikale bildenden Verbindungen, sogenannten Initiatoren. Die Initiatoren werden üblicherweise in Mengen bis zu 10 Gew.-%, vorzugsweise 0,05 bis 15 Gew.-% und insbesondere 0,2 bis 5 Gew.-%, bezogen auf die zu polymerisierenden Monomere M + M', eingesetzt. Bei aus mehreren Bestandteilen bestehenden Initiatoren (Initiatorsysteme, z. B. bei Redox-Initiatorsystemen) beziehen sich die vorstehenden Gewichtsangaben auf die Summe der Komponenten.

Geeignete Initiatoren sind beispielsweise organische Peroxide und Hydroperoxide, weiterhin Peroxodisulfate, Percarbonate, Peroxidester, Wasserstoffperoxid und Azoverbindungen. Beispiele für Initiatoren sind Wasserstoffperoxid, Dicyclohexylperoxidicarbonat, Diacetylperoxid, Di-tert.-butylperoxid, Diamylperoxid, Dioctanoylperoxid, Didecanoylperoxid, Dilauroylperoxid, Dibenzoylperoxid, Bis(o-toluyl)peroxid, Succinylperoxid, Methylethylketonperoxid, Di-tert.-butylhydroperoxid, Acetylacetonperoxid, Butylperacetat, tert.-Butylpermaleinat, tert.-Butylperisobutyrat, tert.-Butylperpivalat, tert.-Butylperoctoat, tert.-Butylperneodecanoat, tert.-Butylperbenzoat, tert.-Butylhydroperoxid, Cumolhydroperoxid, tert.-Butylperneodecanoat, tert.-Amylperpivalat, tert.-Butylperpivalat, tert.-Butylperbenzoat, tert.-Butylperoxi-2-ethylhexanoat und Diisopropylperoxidicarbamat; weiterhin Lithium-, Natrium-, Kalium- und Ammoniumperoxodisulfat, 2,2'-Azobis-isobutyronitril, 2,2'-Azobis(2-methylbutyronitril), 2,2'-Azobis[2-methyl-N-(-2-hydroxyethyl)]propionamid, 1,1'-Azobis(1-cyclohexancarbonitril), 2,2'-Azobis(2,4-dimethylvaleronitril), 2,2'-Azobis(N,N'-dimethylenisobutyroamidin)dihydrochlorid, und 2,2'-Azobis(2-amidinopropan)dihydrochlorid, sowie die im Folgenden erläuterten Redox-Initiatorsysteme.

Redox-Initiatorsysteme umfassen mindestens eine oxidierende, in der Regel eine Peroxid-Verbindung, und wenigstens eine reduzierend wirkende Verbindung, beispielsweise eine reduzierend wirkende Schwefelverbindung wie Bisulfite, Sulfite, Thiosulfate, Dithionite, Tetrathionate von Alkalimetallen oder deren Ammoniumsalze oder ein organisches Reduktionsmittel wie Benzoin, Dimethylanilin, Ascorbinsäure, Hydroxymethansulfinate sowie Addukte von Hydrogensulfit an Ketone wie beispielsweise das Aceton-Bisulfit-Addukt.

In Kombination mit den Initiatoren bzw. den Redox-Initiatorsystemen können zusätzlich Übergangsmetallkatalysatoren eingesetzt werden, z. B. Salze von Eisen, Kobalt, Nickel, Kupfer, Vanadium und Mangan. Geeignete Salze sind z. B. Eisen(II)sulfat, Kobalt (II)chlorid, Nickel(II)sulfat, oder Kupfer(I)chlorid. Bezogen auf die Monomere, wird das reduzierend wirkende Übergangsmetallsalz in einer Konzentration von 0,1 ppm bis 1000 ppm eingesetzt. So kann man Kombinationen von Wasserstoffperoxid mit Eisen(II)-Salzen einsetzen, wie beispielsweise 0,5 bis 30 % Wasserstoffperoxid und 0,1 bis 500 ppm Mohrsches Salz.

Bevorzugte Initiatoren sind organische Peroxide und Hydroperoxide sowie Redox-Initiatoren, die eines der vorgenannten organischen Peroxide oder Hydroperoxide umfassen.

Gegebenenfalls kann es erforderlich sein, das Molekulargewicht der herzustellenden Polymere zu kontrollieren. Hierzu wird man in der Regel die Polymerisation der Monomere M in Gegenwart von Reglern durchführen. Zu den Reglern zählen beispielsweise organische SH-Gruppen enthaltende Verbindungen, insbesondere wasserlösliche SH-Gruppen enthaltende Verbindungen wie 2-Mercaptoethanol, 2-Mercaptopropanol, 3-Mercaptopropionsäure, Cystein und N-Acetylcystein. Die Polymerisationsregler werden, sofern erwünscht, im Allgemeinen in Mengen von 0,05 bis 2 Gew.-%, insbesondere 0,1 bis 1 Gew.-%, bezogen auf die Monomere M, eingesetzt.

Erfindungsgemäß erfolgt die Polymerisation der Monomere M in einem organischen Lösungsmittel, das mit Wasser mischbar ist, oder in einer Mischung, die wenigstens ein mit Wasser mischbares organisches Lösungsmittel und Wasser umfasst. In diesen Mischungen beträgt der Anteil an Wasser vorzugsweise nicht mehr als 50 Gew.-%, z. B. 1 bis 50 Gew.%, insbesondere nicht mehr als 40 Gew.-%, z. B. 2 bis 40 Gew.-%, besonders bevorzugt nicht mehr als 30 Gew.-%, z. B. 5 bis 30 Gew.-%, bezogen auf die Gesamtmenge an Wasser plus organischem Lösungsmittel.

Hier und im Folgenden werden als mit Wasser mischbare Lösungsmittel solche organische Lösungsmittel bezeichnet, die bei 25 °C und 1 bar mit 50 Gewichtsteilen Wasser, bezogen auf 100 Gewichtsteile organisches Lösungsmittel, eine homogene Phase bilden. Hierzu zählen vorzugsweise protische Lösungsmittel wie C₁-C₄-Alkanole, z. B. Methanol, Ethanol, n-Propanol, Isopropanol, n-Butanol, cyclische Ether wie Tetrahydrofuran und Dioxan, Ketone wie Aceton, Methylethylketon und Cyclohexanon, Glykole und Glykolether wie Ethylenglykol, Propylenglykol, Ethylenglykolmonomethylether, Diethylenglykol, Triethylenglykol, Diethylenglykolmonomethylether und Triethylenglykolmonomethylether. Bevorzugte organische Lösungsmittel sind C₁-C₄-Alkanole. Insbesondere führt man das erfindungsgemäße Verfahren in einem C₁-C₄-Alkanol oder in einer Mischung eines C₁-C₄-Alkanols mit Wasser in den oben genannten Mengenverhältnissen durch.

Erfindungsgemäß erfolgt gegen Ende der Polymerisation die Zugabe der Monomerzusammensetzung M'. Das bedeutet, dass die Zugabe der Monomerzusammensetzung M' in der Regel zu einem Zeitpunkt erfolgt, wenn wenigstens 80 %, insbesondere wenigstens 90 % und besonders bevorzugt wenigstens 95 % desjenigen Anteils der Monomere M, der von den Monomeren M' verschieden ist, also nicht zur Monomerzusammensetzung M' gehört, sich bereits im Polymerisationsgefäß unter Polymerisationsbedingungen befindet. Bei einem Monomerzulaufverfahren ist dies in der Fall, wenn wenigstens 80 %, vorzugsweise wenigstens 90 % und insbesondere wenigstens 95 % dieses Monomeranteils dem Polymerisationsgefäß zugführt wurden. Insbesondere erfolgt die Zugabe der Monomerzusammensetzung M' und ihre Polymerisation, wenn sich bereits die Gesamtmenge dieses Monomeranteils im Polymerisationsgefäß befinden, im Falle einer Polymerisation nach einem Monomerzulaufverfahren nach Beendigung der Zugabe dieses Monomeranteils. Die Zugabe der Monomerzusammensetzung M' kann in einer Portion, in mehreren Portionen oder kontinuierlich über einen längeren Zeitraum erfolgen. Vorzugsweise erfolgt die Zugabe der Monomerzusammensetzung M' über einen Zeitraum von 10 Minuten bis 1 h.

Während oder im Anschluss an die Zugabe der Monomerzusammensetzung M' erfolgt die Polymerisation der in der Monomerzusammensetzung M' enthaltenen Monomere. Die Polymerisation der Monomere M' wird wie bereits die Polymerisation der Monomere M durch einen Polymerisationsinitiator ausgelöst. Geeignete Polymerisationsinitiatoren für die Polymerisation der Monomere M' sind die zuvor genannten Initiatoren und insbesondere die dort als bevorzugt genannten Polymerisationsinitiatoren.

Für die Polymerisation des von den Monomeren M' verschiedenen Anteils der Monomere M hat es sich bewährt, nach einem Monomerzulaufverfahren zu arbeiten. Hierbei führt man die überwiegende Menge der zu polymerisierenden Monomere, vorzugsweise wenigstens 70 Gew.-%, z. B. 70 bis 99 Gew.-% und insbesondere wenigstens 80 Gew.-%, z. B. 80 bis 95 Gew.-% der zu polymerisierenden Monomere als Monomerzulauf der Polymerisationsreaktion in ihrem Verlauf zu. Grundsätzlich kann der Monomerzulauf die Gesamtmenge der von den Monomeren M' verschiedenen Monomere enthalten. Vorzugsweise legt man jedoch etwa 1 bis 30 Gew.-% und insbesondere 2 bis 20 Gew.-% der zu polymerisierenden Monomere M im Reaktionsgefäß vor. Zum Starten der Polymerisation erwärmt man die Vorlage auf Polymerisationstemperatur und beginnt dann mit der Zugabe der Restmenge der Monomere und der Restmenge an Polymerisationsinitiator. Die Zugabe der Monomere sowie des Polymerisationsinitiators ist in der Regel zeitlich aufeinander abgestimmt und kann portionsweise, in Intervallen oder kontinuierlich mit gleichbleibender oder veränderlicher Zulaufrate erfolgen. Die Zugabe der Monomere kann sowohl in Form der reinen Monomere als auch in einer Lösung dieser Monomere in dem organischen Lösungsmittel oder in der Mischung aus organischem Lösungsmittel und Wasser erfolgen. Die Konzentration der Monomere im Zulauf liegt üblicherweise im Bereich von 10 bis 70 Gew.-% und insbesondere im Bereich von 30 bis 60 Gew.-%, bezogen auf die Gesamtmenge des Monomerzulaufs.

Die für die Polymerisation der Monomere M erforderliche Reaktionstemperatur liegt üblicherweise im Bereich von 20 bis 150 °C und insbesondere im Bereich von 50 bis 110 °C, abhängig von dem jeweils eingesetzten Initiatorsystem. Der Reaktionsdruck ist von untergeordneter Bedeutung und liegt üblicherweise im Bereich von 800 mbar bis 1,2 bar. In der Regel wird man die Reaktion bei Umgebungsdruck oder unter dem bei der Polymerisation herrschenden Dampfdruck des Reaktionsgemischs durchführen. Vorzugsweise führt man die Polymerisation in einer inerten Atmosphäre, beispielsweise unter Argon oder einem Stickstoffstrom, durch.

Der die Polymerisation auslösende Initiator kann im Polymerisationsgefäß vorgelegt werden und wird vorzugsweise zumindest teilweise im Verlauf der Polymerisation zugegeben. Die Zugabe des Polymerisationsinitiators kann portionsweise, in Intervallen oder kontinuierlich mit gleichbleibender oder veränderlicher Zulaufrate erfolgen. Vorzugsweise erfolgt die Zugabe des Polymerisationsinitiators in Form einer Lösung in dem organischen Lösungsmittel oder in einer Mischung des organischen Lösungsmittels mit Wasser. Während der Polymerisation wird man üblicherweise Lösungsmittel bzw. Lösungsmittel/Wasser in einer Menge verwenden, dass die Konzentration an Monomer bzw. Monomer plus Polymer im Polymerisationsgefäß im Bereich von 10 bis 70 Gew.-% und insbesondere 30 bis 60 Gew.-%, bezogen auf die Gesamtmenge an Polymer, Monomer, organischem Lösungsmittel und gegebenenfalls Wasser, liegt.

Um eine Polymerisation der Monomere M' zu gewährleisten, wird man die Polymerisationsbedingungen naturgemäß so wählen, dass zum Zeitpunkt der Zugabe der Monomerzusammensetzung M' noch unverbrauchter Polymerisationsinitiator im Polymerisationsgefäß vorliegt oder man wird die erforderliche Menge an Polymerisationsinitiator mit den Monomeren M' zugeben. Vorzugsweise wird man so vorgehen, dass der überwiegende Anteil des Polymerisationsinitiators, vorteilhafterweise wenigstens 80% und insbesondere wenigstens 90 %, über einen längeren Zeitraum unter Polymerisationsbedingungen dem Polymerisationsgefäß zugeführt wird. Insbesondere wird man die Zugaberate so wählen, dass sich zum Zeitpunkt der Zugabe der Monomere M' 1 bis 50 %, insbesondere 2 bis 30 % der Gesamtmenge an Polymerisationsinitiator noch nicht im Polymerisationsgefäß befinden. Insbesondere wird man so vorgehen, dass die Beendigung der Initiatorzugabe nicht vor Beendigung der Zugabe der Monomerzusammensetzung M' erfolgt.

Gegebenenfalls kann sich der Polymerisation der Monomere M' eine Nachpolymerisation anschließen, d.h. die Polymerisationsbedingungen werden nach Beendigung der Zugabe der Monomere M' noch eine Zeit lang aufrecht erhalten, z.B. für einen Zeitraum von 10 min. bis 5 h.

Dem Polymerisationsverfahren kann sich außerdem eine chemische oder physikalische Desodorierung anschließen. In der Regel wird man jedoch auf eine Desodorierung verzichten, da beim Entfernen des Lösungsmittels die überwiegende Menge an nicht polymerisierten Monomeren und sonstigen flüchtigen Verunreinigungen ebenfalls entfernt werden.

Auf diese Weise erhält man ein lösungsmittelhaltiges Polymerisationsgemisch. Erfindungsgemäß schließt sich dann die Entfernung des organischen Lösungsmittels und der Ersatz des Lösungsmittels durch Wasser an. Der Ersatz des organischen Lösungsmittels durch Wasser kann nach den hierfür üblichen Methoden erfolgen, beispielsweise indem man das organische Lösungsmittel auf destillativem Wege entfernt und sukzessive oder kontinuierlich durch Wasser ersetzt. Auf diese Weise erhält man die erfindungsgemäßen, wässrigen Sekundärdispersionen der nach diesem Verfahren hergestellten wasserunlöslichen Polymere.

Die nach dem erfindungsgemäßen Verfahren erhältlichen wässrigen Sekundärdispersionen zeichnen sich durch eine hohe Stabilität gegenüber Sedimentation und Koagulation auch nach längerer Lagerung, beispielsweise nach einer Lagerung von vier bis sechs Wochen oder bei Lagerung bei erhöhter Temperatur, aus. Sie sind daher ebenfalls Gegenstand der vorliegenden Erfindung.

Die wässrigen Sekundärdispersionen enthalten üblicherweise das nach dem erfindungsgemäßen Verfahren erhältliche Copolymer in einer Menge von 5 bis 60 Gew.-%, insbesondere in einer Menge von 10 bis 30 Gew.-%.

Der K-Wert (nach Fikentscher - Cellulosechemie 1932, Bd. 13, S. 58-64 und S. 71-74) der erfindungsgemäß erhältlichen Copolymere liegt typischerweise im Bereich von 20 bis 100 und insbesondere im Bereich von 20 bis 80 (bestimmt als Lösung in Ethanol oder Isopropanol bei 25 °C bei einer Konzentration, abhängig vom K-Wert, im Bereich von 0,1 bis 5 Gew.-%).

Auf Grund der verbesserten Stabilität gegenüber Sedimentation und Koagulation ist es nicht erforderlich, dass man während der Herstellung der wässrigen Polymerdispersion grenzflächenaktive Verbindungen wie Emulgatoren oder Schutzkolloide zusetzt. Daher wird man in einer bevorzugten Ausführungsform der Erfindung keine oder weniger als 0,5 Gew.-%, bezogen auf das Gesamtgewicht der Monomere M, grenzflächenaktive Verbindungen zusetzen

Auf Grund der verbesserten Stabilität gegenüber Sedimentation und Koagulation lassen sich die erfindungsgemäßen wässrigen Sekundärdispersionen leichter in ein Pulver überführen. Gegenstand der Erfindung sind daher auch Polymerpulver, die durch Trocknen der erfindungsgemäßen wässrigen Sekundärdispersionen, d.h. durch Entfernen des Wassers und sonstiger flüchtiger Komponenten der Sekundärdisperionen erhältlich sind.

Die mit der Trocknung von lösungsmittelhaltigen Polymerisationsgemischen naturgemäß verbundenen Sicherheitsrisiken und technischen Probleme treten bei der Trocknung der erfindungsgemäßen wässrigen Sekundärdispersion nicht auf.

Die Trocknung der wässrigen Sekundärdisperisonen kann in üblicher Weise erfolgen, z.B. durch Sprühtrocknung, Gefriertrocknung, Wirbelschichttrocknung, Trocknung im Extruder und Walzentrocknung. Gegebenenfalls gibt man beim Trocknen in geringer Menge, z.B. 0,05 bis 2 Gew.-%, insbesondere 0,1 bis 1 Gew.-%, bezogen auf das zu trocknende Polymerisat, Sprührtrocknungshilfsmittel zu, die ein Verklumpen des Polymerisats verhindern oder vermindern. Geeignete Sprühtrocknungshilfsmittel sind insbesondere Kieselsäuren, speziell pyrogene Kieselsäuren, die gegebenenfalls hydrophobiert sind.

Die erfindungsgemäß erhältlichen Sekundärdispersionen und die darin enthaltenen Polymerisate sind für viele Anwendungszwecke, insbesondere zur Herstellung pharmazeutischer oder kosmetischer Zubereitungen geeignet. Dies gilt insbesondere für die Copolymere der Monomere M1a mit M2 sowie gegebenenfalls mit M3, die beispielsweise zur Herstellung von kosmetischen Wasser-in-Öl-Emulsionen wie beschrieben in DE-A 2514100, als Verdicker für hydrophobe Flüssigkeiten wie beschrieben in DE 10108387 sowie zur Herstellung von festen kosmetischen und pharmazeutischen Darreichungsformen wie beschrieben in EP-A 953358 oder WO 99/27916, geeignet sind.

Die folgenden Beispiele sollen die Erfindung verdeutlichen, ohne sie jedoch einzuschränken.

### Beispiel 1: Herstellung einer wässrigen Sekundärdispersion durch Polymerisation von N-Vinylpyrrolidon mit Stearylmethacrylat im Gewichtsverhältnis 7:3 in n-Propanol

In einem Polymerisationsgefäß legte man 17 g Stearylmethacrylat, 39 g N-Vinylpyrrolidon in 100 g n-Propanol vor und erhitzte unter einer Stickstoffatmosphäre auf 77 °C Innentemperatur. Dann gab man unter Rühren und Beibehaltung der Temperatur Zulauf 1 innerhalb von 5,5 h und Zulauf 2 innerhalb von 6,5 h zu. Nach Beendigung von Zulauf 1 begann man mit der Zugabe von Zulauf 3 innerhalb 30 Minuten. Nach Beendigung von Zulauf 2 hielt man die Temperatur weitere 120 Minuten bei. Dann begann man unter Zugabe von Wasser eine Mischung aus Wasser und n-Propanol abzudestillieren, bis der Gehalt an n-Propanol im Destillationssumpf auf < 1 Gew.-% abgesunken war. Auf diese Weise erhielt man eine wässrige Sekundärdispersion mit einem Feststoffgehalt von 21 Gew.-%, die auch nach vier Wochen Lagerung bei Raumtemperatur keine Anzeichen von Sedimentation oder Koagulation zeigte. Der K-Wert des Polymeren (nach Fikentscher s.o., gemessen als 1 %ige Lösung in Ethanol bei 25°C) betrug 45.

Zulauf 1: 195 g Vinylpyrrolidon, 103 g Stearylmethacrylat, 240 g n-Propanol

Zulauf 2:
2,25 g tert.-Butylperoxopivalat
57 g n-Propanol

Zulauf 3:
46 g N-Vinylpyrrolidon
10 g n-Propanol

### Beispiel 2: Herstellung einer wässrigen Sekundärdispersion durch Polymerisation von N-Vinylpyrrolidon mit Stearylmethacrylat im Gewichtsverhältnis 7:3 in n-Propanol

Die Herstellung der wässrigen Sekundärdispersion erfolgte in Analogie zu der Vorschrift aus Beispiel 1, wobei abweichend hierzu Zulauf 1 221 g N-Vinylpyrrolidon, 103 g Stearylmethacrylat und 240 g n-Propanol enthielt und Zulauf 3 die folgende Zusammensetzung aufwies: 20 g N-Vinylpyrrolidon und 10 g n-Propanol. Auf diese Weise erhielt man eine wässrige Sekundärdispersion mit einem Feststoffgehalt von 16 Gew.-%, die auch nach vier Wochen Lagerung bei Raumtemperatur keine Anzeichen von Sedimentation oder Koagulation zeigte. Der K-Wert des Polymeren (nach Fikentscher s.o., gemessen als 1 %ige Lösung in Ethanol bei 25°C) betrug 45.

### Vergleichsbeispiel 1

In Analogie zu der Herstellungsvorschrift in Beispiel 1 stellte man eine wässrige Sekundärdispersion her, wobei abweichend zu der Vorschrift aus Beispiel 1 keine Zugabe eines Zulaufs 3 vorgenommen wurde und Zulauf 1 die folgende Zusammensetzung aufwies: 241 g N-Vinylpyrrolidon, 103 g Stearylmethacrylat und 250 g n-Propanol. Die erhaltene Sekundärdispersion zeigte nach vier Wochen bei 25°C starke Sedimentationserscheinungen. Der K-Wert des Polymeren lag bei 45 Der Feststoffgehalt der Dispersion betrug 15 Gew.-%.

### Vergleichsbeispiel 2

Die Herstellung der wässrigen Sekundärdispersion erfolgte in Analogie zu der Vorschrift aus Beispiel 1, wobei abweichend hierzu Zulauf 3 46 g Polyvinylpyrrolidon mit einem k-Wert von 12 enthielt. Die erhaltene wässrige Sekundärdispersion zeigte nach vier Wochen deutliche Anzeichen von Sedimentation. Der K-Wert des Polymeren lag bei 45. Der Feststoffgehalt der Dispersion betrug 16 Gew.-%.

### Vergleichsbeispiel 3

Die Herstellung der wässrigen Sekundärdispersion erfolgte in Analogie zu der Vorschrift aus Beispiel 1, wobei abweichend hierzu Zulauf 3 46 g Polyvinylpyrrolidon mit einem k-Wert von 17 enthielt. Die erhaltene wässrige Sekundärdispersion zeigte nach vier Wochen deutliche Anzeichen von Sedimentation. Der K-Wert des Polymeren lag bei 44. Der Feststoffgehalt der Dispersion betrug 16 Gew.-%.

### Vergleichsbeispiele 4 bis 5

Die Herstellung der wässrigen Sekundärdispersion erfolgte in Analogie zu der Vorschrift aus Beispiel 2, mit den folgenden Maßgaben:
- In Vergleichsbeispiel 4 enthielt Zulauf 3 20 g Polyvinylpyrrolidon mit einem k-Wert von 12.
- In Vergleichsbeispiel 5 enthielt Zulauf 3 20 g Polyvinylpyrrolidon mit einem k-Wert von 17.

Die erhaltenen wässrigen Sekundärdispersionen zeigten alle nach vier Wochen deutliche Anzeichen von Sedimentation. Der K-Wert des Polymeren lag bei 45 Der Feststoffgehalt der Dispersion betrug 16 Gew.-%.

### Beispiel 3: Herstellung einer wässrigen Sekundärdispersion durch Polymerisation von N-Vinylpyrrolidon mit Stearylmethacrylat im Gewichtsverhältnis 7:3 in n-Propanol/Wasser (8:2 v/v)

In einem Polymerisationsgefäß legte man 5 g N-Vinylpyrrolidon in einer Mischung aus 70 g n-Propanol und 30 g Wasser vor und erhitzte unter einer Stickstoffatmosphäre auf 77 °C Innentemperatur. Dann gab man unter Rühren und Beibehaltung der Temperatur Zulauf 1 innerhalb von 5,5 h und Zulauf 2 innerhalb von 6,5 h zu. Nach Beendigung von Zulauf 1 begann man mit der Zugabe von Zulauf 3 innerhalb 30 Minuten. Nach Beendigung von Zulauf 2 hielt man die Temperatur weitere 120 Minuten bei. Dann begann man unter Zugabe von Wasser eine Mischung aus Wasser und n-Propanol abzudestillieren, bis der Gehalt an n-Propanol im Destillationssumpf auf < 1 Gew.-% abgesunken war. Auf diese Weise erhielt man eine wässrige Sekundärdispersion mit einem Feststoffgehalt von 16 Gew.-%, die auch nach vier Wochen Lagerung bei Raumtemperatur keine Anzeichen von Sedimentation oder Koagulation zeigte. Der K-Wert des Polymeren (nach Fikentscher s.o., gemessen als 1 %ige Lösung in Ethanol bei 25°C) betrug 44.

Zulauf 1: 229 g Vinylpyrrolidon, 120 g Stearylmethacrylat, 200 g n-Propanol, 42 g Wasser

Zulauf 2:
2,85 g tert.-Butylperoxopivalat
46 g n-Propanol

Zulauf 3:
46 g N-Vinylpyrrolidon

8 g Wasser

### Beispiel 4

Die Herstellung der wässrigen Sekundärdispersion erfolgte in Analogie zu der Vorschrift aus Beispiel 3, wobei abweichend hierzu Zulauf 1 255 g N-Vinylpyrrolidon, 120 g Stearylmethacrylat, 200 g n-Propanol und 42 g Wasser enthielt und Zulauf 3 die folgende Zusammensetzung aufwies: 20 g N-Vinylpyrrolidon und 8 g Wasser. Auf diese Weise erhielt man eine wässrige Sekundärdispersion mit einem Feststoffgehalt von 18 Gew.-%, die auch nach vier Wochen Lagerung bei Raumtemperatur keine Anzeichen von Sedimentation oder Koagulation zeigte.

### Vergleichsbeispiel 6

In Analogie zu der Herstellungsvorschrift in Beispiel 3 stellte man eine wässrige Sekundärdispersion her, wobei abweichend zu der Vorschrift aus Beispiel 3 keine Zugabe eines Zulaufs 3 vorgenommen wurde und Zulauf 1 275 g N-Vinylpyrrolidon, 120 g Stearylmethacrylat, 200 g n-Propanol und 50 g Wasser enthielt. Die erhaltene Sekundärdispersion zeigte nach vier Wochen bei 25°C starke Sedimentationserscheinungen. Der K-Wert des Polymeren lag bei 44. Der Feststoffgehalt der Dispersion betrug 14 Gew.-%.

### Vergleichsbeispiel 7

Die Herstellung der wässrigen Sekundärdispersion erfolgte in Analogie zu der Vorschrift aus Beispiel 3, wobei abweichend hierzu Zulauf 3 46 g Polyvinylpyrrolidon mit einem k-Wert von 12 enthielt. Die erhaltene wässrige Sekundärdispersion zeigte nach vier Wochen deutliche Anzeichen von Sedimentation. Der K-Wert des Polymeren lag bei 44. Der Feststoffgehalt der Dispersion betrug 15 Gew.-%.

### Vergleichsbeispiel 8

Die Herstellung der wässrigen Sekundärdispersion erfolgte in Analogie zu der Vorschrift aus Beispiel 3, wobei abweichend hierzu Zulauf 3 46 g Polyvinylpyrrolidon mit einem k-Wert von 17 enthielt. Die erhaltene wässrige Sekundärdispersion zeigte nach vier Wochen deutliche Anzeichen von Sedimentation. Der K-Wert des Polymeren lag bei 44. Der Feststoffgehalt der Dispersion betrug 16 Gew.-%.

### Vergleichsbeispiele 9 bis 10

Die Herstellung der wässrigen Sekundärdispersion erfolgte in Analogie zu der Vorschrift aus Beispiel 4, mit den folgenden Maßgaben:
- In Vergleichsbeispiel 9 enthielt Zulauf 3 20 g Polyvinylpyrrolidon mit einem k-Wert von 12.
- In Vergleichsbeispiel 10 enthielt Zulauf 3 20 g Polyvinylpyrrolidon mit einem k-Wert von 17.

Die erhaltenen wässrigen Sekundärdispersionen zeigten alle nach vier Wochen deutliche Anzeichen von Sedimentation. Der K-Wert des Polymeren lag bei 44. Der Feststoffgehalt der Dispersion betrug 15 Gew.-%.

### Beispiel 5: Herstellung einer wässrigen Sekundärdispersion durch Polymerisation von N-Vinylpyrrolidon mit Stearylmethacrylat im Gewichtsverhältnis 7:3 in n-Propanol/Wasser (6:4 v/v)

Die Herstellung der wässrigen Sekundärdispersion erfolgte nach der Vorschrift des Beispiels 3, wobei das Reaktionsmedium Wasser/n-Propanol im Gewichtsverhältnis 4:6 war. Die erhaltene Sekundärdispersion zeigte nach vier Wochen Lagerung bei Raumtemperatur keine Anzeichen von Sedimentation oder Koagulation. Der K-Wert des Polymeren lag bei 45. Der Feststoffgehalt der Dispersion betrug 18 Gew.-%.

### Vergleichsbeispiele 11 und 12

Die Herstellung der wässrigen Sekundärdispersionen erfolgte nach der Vorschrift der Vergleichsbeispiele 6 bzw. 7, wobei das Reaktionsmedium Wasser/n-Propanol im Gewichtsverhältnis 4:6 war. In allen Dispersionen wurden nach 4 Wochen bei Raumtemperatur starke Koagulation beobachtet. Der K-Wert des Polymeren lag bei 45. Der Feststoffgehalt der Dispersion betrug 16 Gew.-%.

### Beispiel 6: Herstellung einer wässrigen Sekundärdispersion durch Polymerisation von N-Vinylpyrrolidon mit Stearylmethacrylat im Gewichtsverhältnis 6:4 in n-Propanol/Wasser (8:2 v/v).

Die Herstellung der wässrigen Sekundärdispersion erfolgte nach der Vorschrift des Beispiels 3, wobei das Gewichtsverhältnis von N-Vinylpyrrolidon zu Stearylmethacrylat 6:4, bezogen auf die Gesamtmenge von N-Vinylpyrrolidon und Stearylmethacrylat in Vorlage, Zulauf 1 und Zulauf 3, war. Die erhaltene Sekundärdispersion zeigte nach vier Wochen Lagerung bei Raumtemperatur keine Anzeichen von Sedimentation oder Koagulation. Der K-Wert des Polymeren lag bei 45. Der Feststoffgehalt der Dispersion betrug 15 Gew.-%.

### Sprühtrocknung (allgemeine Vorschrift):

Die wässrige Dispersion wurde über eine 1-Stoffdüse in den Gasstrom (Gleichstrom) eines Sprühtrockners eingedüst. Die Gaseintrittstemperatur lag bei 180°C die Gasaustrittstemperatur lag bei 90-95°C. Über eine zweite Düse wurde pyrogene Kieselsäure (Aerosil 200, Degussa) in einer Menge von 0,2-0,4 Gew.-%, bezogen auf den Polymeranteil der Dispersion.

Auf diese Weise erhielt man im Falle der Dispersion aus Beispiel 1 ein trockenes, rieselfähiges Pulver in einer Ausbeute von > 90%.

## Patentansprüche

1. Verfahren zur Herstellung wässriger Sekundärdispersionen von wasserunlöslichen Copolymeren, umfassend
a) radikalische Lösungspolymerisation ethylenisch ungesättigter Monomere M, die
i. 10 bis 90 Gew-%, bezogen auf die Gesamtmonomermenge M, wenigstens eines monoethylenisch ungesättigten Monomers M1 mit einer Wasserlöslichkeit von nicht mehr als 30 g/l bei 25 °C und 1 bar und
ii. 10 bis 90 Gew-%, bezogen auf die Gesamtmonomermenge M, wenigstens eines hydrophilen, monoethylenisch ungesättigten, nichtionischen Comonomeres M2 mit einer Wasserlöslichkeit von wenigstens 80 g/l bei 25 °C und 1 bar umfassen,
in einem organischen, mit Wasser mischbaren Lösungsmittel, das bei 25 °C und 1 bar mit 50 Gewichteilen Wasser, bezogen auf 100 Gewichtsteile organisches Lösungsmittel, eine homogene Phase bildet, oder in einer Mischung von Wasser mit dem organischen, mit Wasser mischbaren Lösungsmittel als Polymerisationsmedium und
b) Ersetzen des organischen Lösungsmittels durch Wasser,
**dadurch gekennzeichnet, dass** man 0,5 bis 20 Gew.-% der Monomere M, bezogen auf die Gesamtmenge der zu polymerisierenden Monomere M, in Form einer Monomerzusammensetzung M', die als Monomere zu mehr als 90 Gew.-% Monomere M2 enthält, zu einem Zeitpunkt zugibt, wenn wenigstens 80% desjenigen Anteils der Monomere M, der von M' verschieden ist, sich bereits im Polymerisationsgefäß unter Polymerisationsbedingungen befindet.

2. Verfahren nach Anspruch 1, **dadurch gekennzeichnet, dass** der in der Monomerzusammensetzung M' zugegebene Anteil der Monomere M2 1 bis 50 Gew.-% der Gesamtmenge der zu polymerisierenden Monomere M2 beträgt.

3. Verfahren nach einem der vorhergehenden Ansprüche, **dadurch gekennzeichnet, dass** die Monomere M' 1 bis 20 Gew.-% der Gesamtmenge der zu polymerisierenden Monomere M ausmachen.

4. Verfahren nach einem der vorhergehenden Ansprüche, **dadurch gekennzeichnet, dass** die Polymerisation der Monomere M nach einem Monomerzulaufverfahren erfolgt.

5. Verfahren nach einem der vorhergehenden Ansprüche, **dadurch gekennzeichnet, dass** die in der Monomerzusammensetzung M' enthaltenen Monomere M2 ausgewählt sind unter N-Vinyllactamen mit 5 bis 8 Ringatomen und N-Vinylamiden aliphatischer Carbonsäuren mit 1 bis 6 C-Atomen.

6. Verfahren nach Anspruch 5, **dadurch gekennzeichnet, dass** das in der Monomerzusammensetzung M' enthaltene Monomer M2 N-Vinylpyrrolidon ist.

7. Verfahren nach einem der vorhergehenden Ansprüche, **dadurch gekennzeichnet, dass** Gesamtmenge der Monomere M,
- 10 bis 60 Gew.-Monomere M1 und
- 40 bis 90 Gew.-% Monomere M2 umfassen.

8. Verfahren nach einem der vorhergehenden Ansprüche, **dadurch gekennzeichnet, dass** die Monomere M1 zu wenigstens 90 Gew.-% wenigstens eines Monomers M1a mit einer Wasserlöslichkeit von nicht mehr als 1 g/l bei 25 °C und 1 bar umfassen.

9. Verfahren nach Anspruch 7 oder 8, **dadurch gekennzeichnet, dass** die Monomere M1a ausgewählt sind unter Estern und Amiden monoethylenisch ungesättigter Carbonsäuren, die wenigstens einen Kohlenwasserstoffrest mit 6 bis 30 C-Atomen aufweisen.

10. Verfahren nach Anspruch 9, **dadurch gekennzeichnet, dass** die Monomere M1a ausgewählt sind unter Estern der Acrylsäure mit C₈-C₂₄-Alkanolen oder C₈-C₂₄-Cycloalkanolen, Estern der Methacrylsäure mit C₈-C₂₄-Alkanolen oder C₈-C₂₄-Cycloalkanolen, den N-C₈-C₂₄-Alkylamiden und den N-C₈-C₂₄-Cycloalkylamiden der Acrylsäure und der Methacrylsäure sowie den N,N-Bis-(C₈-C₂₄-Alkyl)amiden der Acrylsäure und der Methacrylsäure.

11. Verfahren nach einem der vorhergehenden Ansprüche, **dadurch gekennzeichnet, dass** die Monomere M, bezogen auf ihr Gesamtgewicht, keine oder weniger als 0,1 Gew.-% Monomere umfassen, die ionische oder ionisierbare Gruppen aufweisen.

12. Verfahren nach einem der vorhergehenden Ansprüche, **dadurch gekennzeichnet, dass** man während der Herstellung der wässrigen Polymerdispersion keine oder weniger als 0,5 Gew.-%, bezogen auf das Gesamtgewicht der Monomere M, grenzflächenaktive Verbindungen zusetzt.

13. Verfahren nach einem der vorhergehenden Ansprüche, **dadurch gekennzeichnet, dass** man die Polymerisation in einem C₁-C₄-Alkanol oder einer Mischung eines C₁-C₄-Alkanols mit Wasser durchführt.

14. Wässrige Sekundärdispersion von wasserunlöslichen Polymeren, erhältlich nach einem Verfahren gemäß einem der vorhergehenden Ansprüche.

15. Polymerpulver, erhältlich durch Trocknen einer wässrigen Sekundärdispersion von wasserunlöslichen Polymeren nach Anspruch 14.

16. Verwendung einer wässrigen Polymerdispersion nach Anspruch 14 oder eines daraus hergestellten Polymerpulvers in kosmetischen oder pharmazeutischen Zubereitungen.

## Claims

1. A method of producing aqueous secondary dispersions of water-insoluble copolymers, comprising
a) free-radical solution polymerization of ethylenically unsaturated monomers M which comprise
i. 10 to 90% by weight, based on the total amount of monomers M, of at least one monoethylenically unsaturated monomer M1 with a solubility in water of not more than 30 g/l at 25°C and 1 bar and
ii. 10 to 90% by weight, based on the total amount of monomers M, of at least one hydrophilic, monoethylenically unsaturated, nonionic comonomer M2 with a solubility in water of at least 80 g/l at 25°C and 1 bar,
in an organic, water-miscible solvent which forms a homogeneous phase at 25°C and 1 bar with 50 parts by weight of water, based on 100 parts by weight of organic solvent, or in a mixture of water with the organic, water-miscible solvent as polymerization medium and
b) replacement of the organic solvent by water,
wherein, 0.5 to 20% by weight of the monomers M, based on the total amount of the monomers M to be polymerized, in the form of a monomer composition M', which comprises as monomers to more than 90% by weight monomers M2, are added at a time when at least 80% of that fraction of the monomers M that is different from M' is already in the polymerization vessel under polymerization conditions.

2. The method according to claim 1, wherein the fraction of monomers M2 added to the monomer composition M' is 1 to 50% by weight of the total amount of the monomers M2 to be polymerized.

3. The method according to one of the preceding claims, wherein the monomers M' constitute 1 to 20% by weight of the total amount of the monomers M to be polymerized.

4. The method according to one of the preceding claims, wherein the polymerization of the monomers M takes place by a monomer feed method.

5. The method according to one of the preceding claims, wherein the monomers M2 present in the monomer composition M' are chosen from N-vinyllactams having 5 to 8 ring atoms and N-vinylamides of aliphatic carboxylic acids having 1 to 6 carbon atoms.

6. The method according to claim 5, wherein the monomer M2 present in the monomer composition M' is N-vinylpyrrolidone.

7. The method according to one of the preceding claims, wherein the total amount of the monomers M comprise
- 10 to 60% by weight of monomers M1 and
- 40 to 90% by weight of monomers M2.

8. The method according to one of the preceding claims, wherein the monomers M1 comprise to at least 90% by weight at least one monomer M1a with a solubility in water of not more than 1 g/l at 25°C and 1 bar.

9. The method according to claim 7 or 8, wherein the monomers M1a are chosen from esters and amides of monoethylenically unsaturated carboxylic acids which have at least one hydrocarbon radical having 6 to 30 carbon atoms.

10. The method according to claim 9, wherein the monomers M1a are chosen from esters of acrylic acid with C₈-C₂₄-alkanols or C₈-C₂₄-cycloalkanols, esters of methacrylic acid with C₈-C₂₄-alkanols or C₈-C₂₄-cycloalkanols, the N-C₈-C₂₄-alkylamides and the N-C₈-C₂₄-cycloalkylamides of acrylic acid and of methacrylic acid, and the N,N-bis(C₈-C₂₄-alkyl)amides of acrylic acid and of methacrylic acid.

11. The method according to one of the preceding claims, wherein the monomers M, based on their total weight, comprise no or less than 0.1% by weight of monomers which have ionic or ionizable groups.

12. The method according to one of the preceding claims, wherein, during the production of the aqueous polymer dispersion, no or less than 0.5% by weight, based on the total weight of the monomers M, of interface-active compound are added.

13. The method according to one of the preceding claims, wherein the polymerization is carried out in a C₁-C₄-alkanol or a mixture of a C₁-C₄-alkanol with water.

14. An aqueous secondary dispersion of waster-insoluble polymers obtainable by a method as in one of the preceding claims.

15. A polymer powder obtainable by drying an aqueous secondary dispersion of water-insoluble polymers according to claim 14.

16. The use of an aqueous polymer dispersion according to claim 14 or a polymer powder produced therefrom in cosmetic or pharmaceutical preparations.

## Revendications

1. Procédé pour la préparation de dispersions aqueuses secondaires de copolymères insolubles dans l'eau, comprenant
a) une polymérisation radicalaire en solution de monomères éthyléniquement insaturée M, qui comprennent
i. 10 à 90% en poids, par rapport à la quantité totale de monomères M, d'au moins un monomère M1 éthyléniquement monoinsaturé présentant une solubilité dans l'eau qui n'est pas supérieure à 30 g/l à 25°C et à 1 bar et
ii. 10 à 90% en poids, par rapport à la quantité totale de monomères M, d'au moins un comonomère M2 hygrophile, éthyléniquement monoinsaturé, non ionique présentant une solubilité dans l'eau d'au moins 80 g/l à 25°C et à 1 bar,
dans un solvant organique miscible à l'eau, qui forme une phase homogène à 25°C et à 1 par avec 50 parties en poids d'eau, par rapport à 100 parties en poids de solvant organique ou dans un mélange d'eau avec le solvant organique, miscible à l'eau comme milieu de polymérisation et
b) remplacement du solvant organique par de l'eau, **caractérisé en ce qu'**on ajoute 0,5 à 20% en poids des monomères M, par rapport à la quantité totale des monomères M à polymériser, sous forme d'une composition de monomères M', qui contient comme monomères, plus de 90% en poids de monomères M2, à un moment où au moins 80% de la proportion de monomères M, qui sont différents de M', se trouvent déjà dans le récipient de polymérisation dans des conditions de polymérisation.

2. Procédé selon la revendication 1, **caractérisé en ce que** la proportion de monomères M2 ajoutée dans la composition de monomères M' représente 1 à 50% en poids de la quantité totale des monomères M2 à polymériser.

3. Procédé selon l'une quelconque des revendications précédente, **caractérisé en ce que** les monomères M' représentant 1 à 20% en poids de la quantité totale des monomères M à polymériser.

4. Procédé selon l'une quelconque des revendications précédentes, **caractérisé en ce que** la polymérisation des monomères M a lieu selon un procédé d'alimentation de monomères.

5. Procédé selon l'une quelconque des revendications précédentes, **caractérisé en ce que** les monomères M2 contenus dans la composition de monomères M' sont choisis parmi les N-vinyllactames comprenant 5 à 8 atomes de cycle et les N-vinylamides d'acides carboxyliques aliphatiques comprenant 1 à 6 atomes de carbone.

6. Procédé selon la revendication 5, **caractérisé en ce que** le monomètre M2 contenu dans la composition de monomères M' est la N-vinyipyrrolidone.

7. Procédé selon l'une quelconque des revendications précédentes, **caractérisé en ce que** la quantité totale des monomères M comprend
- 10 à 60% en poids de monomères M1 et
- 40 à 90% en poids de monomères M2.

8. Procédé selon l'une quelconque des revendications précédentes, **caractérisé en ce que** les monomères M1 comprennent au moins 90% en poids d'au moins un monomètre M1a présentant une solubilité dans l'eau qui n'est pas supérieure à 1 g/l à 25°C et à 1 bar.

9. Procédé selon la revendications 7 ou 8, **caractérisé en ce que** les monomères M1a sont choisis parmi les esters et les amides d'acides carboxyliques éthyléniquement monoinsaturés qui présentent au moins un radical hydrocarboné comprenant 6 à 30 atomes de carbone.

10. Procédé selon la revendication 9, **caractérisé en ce que** les monomères M1a sont choisis parmi les esters de l'acide acrylique avec des C₈-C₂₄-alcanols ou des C₈-C₂₄-cycloalcanols, des esters de l'acide méthacrylique avec des C₈-C₂₄-alcanols ou des C₈-C₂₄-cycloalcanols, les N-C₈-C₂₄-alkylamides et les N-C₈-C₂₄-cycloalkylamides de l'acide acrylique et de l'acide méthacrylique ainsi que les N,N-bis-(C₈-C₂₄-alkyl)amides de l'acide acrylique et de l'acide méthacrylique.

11. Procédé selon l'une quelconque des revendications précédentes, **caractérisé en ce que** les monomères M, par rapport à leur poids total, ne présentent pas de monomères ou moins de 0,1% en poids de monomères qui présentent des groupes ioniques ou ionisables.

12. Procédé selon l'une quelconque des revendications précédentes, **caractérisé en ce que**, pendant la préparation de la dispersion aqueuse de polymère, on n'ajoute pas ou moins de 0,5% en poids de composés tensioactifs, par rapport au poids total des monomères M.

13. Procédé selon l'une quelconque des revendications précédentes, **caractérisé en ce qu'**on réalise la polymérisation dans un C₁-C₄-alcanol ou un mélange d'un C₁-C₄-alcanol avec de l'eau.

14. Dispersion aqueuse secondaire de polymères insolubles dans l'eau, pouvant être obtenue selon un procédé selon l'une quelconque des revendications précédentes.

15. Poudre de polymère, pouvant être obtenue par séchage d'une dispersion aqueuse secondaire de polymères insolubles dans l'eau selon la revendication 14.

16. Utilisation d'une Dispersion aqueuse de polymères selon la revendication 14 ou d'une poudre de polymère préparée à partir de celle-ci dans des préparations cosmétiques ou pharmaceutiques.
